# EUROPEAN PATENT APPLICATION

(11) **EP 4 268 903 A1**
(43) Date of publication of application: **01.11.2023**
(21) Application number: 22170700.3
(22) Date of filing: 29.04.2022
(51) Int. Cl.: A61Q 1/10, A61K 8/87, A61K 8/37

(54) **COSMETIC COMPOSITION WITH A BIO-BASED POLYURETHANE DISPERSION**

(71) Applicant: Covestro Deutschland AG, 51373 Leverkusen (DE)
(72) Inventor: The designation of the inventor has not yet been filed
(74) Representative: Levpat

(57) **Abstract**

The present invention relates to a cosmetic composition, especially mascara, the preparation method and use thereof. The cosmetic composition comprises at least an aqueous bio-based polyurethane dispersion; at least a bis-diglyceryl polyacyladipate-2; at least a wax with a melting point not higher than 65°C; at least a wax with a melting point higher than 65°C; and optionally, a cosmetically acceptable medium; wherein the aqueous bio-based polyurethane dispersion has a solid content of 3% by weight to 9% by weight, and the bis-diglyceryl polyacyladipate-2 is in an amount of 3% by weight to 9% by weight, and the wax with a melting point not higher than 65°C and the wax with a melting point higher than 65°C have a weight sum of 4% by weight to 10% by weight and a weight ratio of 0.1 to 1.5, the above weight percentages being based on the total weight of the cosmetic composition.

## Description

### Technical Field

The present invention relates to a cosmetic composition, the preparation method and use thereof.

### Prior art

Mascaras belong to an important part of daily makeup. Mascara may be divided into water-washable and anhydrous formulations. In terms of safety, the volatile solvents in the anhydrous formulation are replaced by the water in the water-washable formulations, which greatly reduces the irritation of the mascaras and improves the safety. In terms of smudge resistance, it is usually necessary to add a high proportion of solid oil into anhydrous formulations, which is prone to smudging, while the film-forming agent in the water-washable formulations may form a hydrophobic and lipophobic solid film and the smudging is improved thereby. In terms of easy removal, water-washable formulations can be easily removed with warm water, which avoids the step of using an oil-soluble makeup remover necessary for anhydrous formulations. Therefore, the water-washable formulation are widely favored by consumers.

To ensure the high volume of eyelashes, it is necessary to add a larger amount of film-forming agent or wax into the water-washable mascaras, which will result in a lack of good curling for the brushed lashes and cause adhesion between the eyelashes, especially visible caking at the tip of the eyelashes, commonly known as "flies' legs" during the brushing process. Therefore, it is desired to develop a water-washable mascara, which not only gives good curling to eyelashes, but also prevent the eyelashes from caking.

US9072686B2 discloses a mascara composition comprising at least one polyurethane composition and at least one hard wax to provide comfort upon brushing, improved volume and curling, wherein the content of the hard wax is at least 12% by weight or more.

CN 104994837 A discloses a composition comprising a latex film former and comprising a wax dispersion and a thermo-reversible gelling polymer.

CN101039719A discloses a emulsion mascara comprising an imide polymer having film forming properties, a wax, water, and a pigment or colorant.

### Summary of the invention

The present invention relates to a cosmetic composition, especially a mascara, the preparation method and use thereof.

The cosmetic composition according to the present invention comprises the following components:
a) at least an aqueous bio-based polyurethane dispersion;
b) at least a bis-diglyceryl polyacyladipate-2;
c) at least a wax with a melting point not higher than 65°C;
d) at least one wax with a melting point higher than 65°C; and
e) optionally, a cosmetically acceptable medium;
wherein the aqueous bio-based polyurethane dispersion has a solid content of 3% by weight to 9% by weight, and the bis-diglyceryl polyacyladipate-2 is in an amount of 3% by weight to 9% by weight, and the wax with a melting point not higher than 65°C and the wax with a melting point higher than 65°C have a weight sum of 4% by weight to 10% by weight and a weight ratio of 0.1 to 1.5, the above weight percentages being based on the total weight of the cosmetic composition.

One aspect of the present invention is to provide a method for preparing the cosmetic composition according to the present invention, including mixing component a) aqueous bio-based polyurethane dispersion, component b) bis-diglyceryl polyacyladipate-2, component c) wax with a melting point not higher than 5°C, component d) wax with a melting point higher than 65°C and component e) optionally a cosmetically acceptable medium to obtain the cosmetic composition.

Another aspect of the present invention is to provide use of the cosmetic composition according to the present invention for applying to keratin fibers for the care or make-up thereof.

Still another aspect of the present invention is to provide a mascara comprising the cosmetic composition according to the present invention.

The cosmetic composition of the present invention comprises components such as the aqueous bio-based polyurethane dispersion, and has good safety, smudge resistance and easy removal. The cosmetic composition of the present invention has suitable adhesiveness and smoothness, and can wrap keratin fibers, especially eyelashes one by one, so as to enhance the volume without losing a natural makeup effect having no caking and having high curling. The cosmetic composition of the present invention has good instant curling and long-lasting curling properties.

### Embodiments

The present invention provides a cosmetic composition, comprising: a) at least an aqueous bio-based polyurethane dispersion; b) at least a bis-diglyceryl polyacyladipate-2; c) at least a wax with a melting point not higher than 65°C; d) at least a wax with a melting point higher than 65°C; and e) optionally, a cosmetically acceptable medium; wherein the aqueous bio-based polyurethane dispersion has a solid content of 3% by weight to 9% by weight, and the bis-diglyceryl polyacyladipate-2 is in an amount of 3% by weight to 9% by weight, and the wax with a melting point not higher than 65°C and the wax with a melting point higher than 65°C have a weight sum of 4% by weight to 10% by weight and a weight ratio of 0.1 to 1.5, the above weight percentages being based on the total weight of the cosmetic composition. The present invention also provides a method for preparing the cosmetic composition and use thereof.

The term "keratin fibers" refer to eyelashes, eyebrows, body hairs or head hairs and imitations of eyelashes, eyebrows, body hairs or hairs.

The term "mascara" is a general term for eyelash cosmetic compositions, eyelash cosmetic primers, eyelash cosmetic top layers, and eyelash care compositions for makeups.

### Component a) aqueous bio-based polyurethane dispersion

The term "aqueous bio-based polyurethane dispersion" refers to the definition of naturally derived ingredients in accordance with the international standard ISO 16128-1:2016, that is, a dispersion having carbon atoms of natural origin accounting for not less than 50% of all carbon atoms in the dispersion.

In the context of the present invention, the term "water-insoluble, water-non-dispersible isocyanate-functionalized polyurethane prepolymer" means that the prepolymer has a solubility in water of less than 10 g/liter at 23°C and that the prepolymer cannot form a sedimentation-stable dispersion in water (especially deionized water) at 23°C. In other words, the prepolymer precipitates out in experiments where it is dispersed in water.

The aqueous bio-based polyurethane dispersion can be added to the cosmetic composition in the form of solid or of dispersion, preferably be added to the cosmetic composition in the form of dispersion, that is, be added to the cosmetic composition in the form of an aqueous polyurethane dispersion. The bio-based polyurethane added to the cosmetic composition in the form of solid can be dispersed in water in the cosmetic composition to form an aqueous bio-based polyurethane dispersion.

For the form of dispersion, the solid weight of the aqueous polyurethane dispersion, i.e. the weight of the effective ingredients of the aqueous polyurethane dispersion = the weight of the dispersion x the solid content of the dispersion. For the form of solid containing crystal water, the solid weight of the aqueous polyurethane dispersion, i.e. the weight of the effective ingredients of the aqueous polyurethane dispersion = the weight of the solid - the weight of the crystal water.

The aqueous bio-based polyurethane dispersion has a solid content of preferably of 4% by weight to 8% by weight, based on the total weight of the cosmetic composition.

The aqueous bio-based polyurethane dispersion is preferably an anionic aqueous bio-based polyurethane dispersion.

The anionic aqueous bio-based polyurethane dispersion is preferably obtained by reacting a system comprising at least a water-insoluble, water-non-dispersible isocyanate-functionalized polyurethane prepolymer a1) and at least an isocyanate-reactive compound a2).

The anionic aqueous bio-based polyurethane dispersion comprises at least a sulfonic acid group and/or sulfonate salt group, most preferably at least a sodium sulfonate group.

The anionic aqueous bio-based polyurethane dispersion has a number-average molecular weight of preferably 4000 g/mol to 8000 g/mol.

### Water-insoluble, water-non-dispersible isocyanate-functionalized polyurethane prepolymer a1)

The water-insoluble, water-non-dispersible isocyanate-functionalized polyurethane prepolymer preferably has terminal isocyanate groups, i.e., the isocyanate groups are located at the chain ends of the prepolymer, and most preferably, all chain ends of the polyurethane prepolymer have isocyanate groups.

The water-insoluble, water-non-dispersible isocyanate-functionalized polyurethane prepolymer is substantially free of ionic groups and/or ionogenic groups.

The water-insoluble, water-non-dispersible, isocyanate-functionalized polyurethane prepolymer can be prepared by reacting a polyester polyol and a polyisocyanate having a glass transition temperature of at least -50°C.

### Isocvanate-reactive compound a2)

The isocyanate-reactive compound is preferably an amino-functional compound.

The amino-functional compound is preferably one or more of the following: primary amines, secondary amines and diamines, most preferably diamines.

The amino-functional compound preferably comprises an amino-functional compound without an ionic group or ionogenic group and an amino-functional compound with an ionic group or ionogenic group.

The amino-functional compound without an ionic group or ionogenic group is preferably one or more of the following: organic diamines, polyamines, compounds containing primary and secondary amino groups, compounds containing a (primary or secondary) amino group and a hydroxyl group, and monofunctional isocyanate-reactive amine compounds.

The organic diamine is preferably one or more of the following: 1,2-ethylene diamine, 1,2- and 1,3-diaminopropane, 1,4-diaminobutane, 1,6-diaminohexane, isophorone diamine, isomer mixtures of 2,2,4- and 2,4,4-trimethylhexamethylene diamine, 2-methylpentamethylene diamine, 4 , 4-diaminodicyclohexyl methane, hydrazine hydrate and dimethylethylene diamine.

The polyamine is preferably diethylene triamine.

The compound containing primary and secondary amino groups is preferably one or more of the following: diethanolamine, 3-amino-1-methylaminopropane, 3-amino-1-ethylaminopropane, 3-amino-1-cyclohexylaminopropane and 3-amino-1-methylaminobutane.

The compound containing a (primary or secondary) amino group and a hydroxyl group, i.e. alkanolamine is preferably one or more of the following: N-aminoethylethanolamine, ethanolamine, 3-aminopropanol and neopentanolamine.

The monofunctional isocyanate-reactive amine compound is preferably one or more of the following: methylamine, ethylamine, propylamine, butylamine, octylamine, laurylamine, stearylamine, isononyloxypropylamine, dimethylamine, diethylamine, dipropylamine, dibutylamine, N-methylaminopropylamine, diethyl(methyl)aminopropylamine, morpholine, piperidine and suitable substituted derivatives thereof.

The amino-functional compound without an ionic group or ionogenic group is most preferably a diamine without an ionic group or ionogenic group.

The amino-functional compound with an ionic group or ionogenic group is preferably an anionic hydrophilizing compound.

The anionic hydrophilizing compound is preferably one or more of the following: 2-(2-aminoethylamino)ethanesulfonic acid, ethylenediamine-propyl- or -butyl-sulfonic acid, 1,2- or 1,3-propanediamine-β-ethylsulfonic acid and taurine and salts thereof, and further preferably those containing a sulfonate salt group as the ionic group and containing two amino groups, most preferably one or more of the following: 2-(2-aminoethylamino)ethanesulfonate salts and 1,3-propanediamine-β-ethanesulfonate salts.

The anionic aqueous bio-based polyurethane dispersion is preferably obtained by the reaction of succinic acid, 1,4-butanediol, neopentyl glycol and isophorone diisocyanate to form a prepolymer, and further the reaction of the prepolymer with sodium N-(2-aminoethyl)-2-aminoethanesulfonate and isophorone diamine.

The anionic aqueous bio-based polyurethane dispersion is most preferably one or more of the following: INCI (International Nomenclature Cosmetic Ingredient) polyurethane-93 and polyurethane-99.

### Preparation of the anionic aqueous bio-based polyurethane dispersion

The process for preparing the anionic aqueous bio-based polyurethane dispersion is as follows: preparing the water-insoluble, water-non-dispersible isocyanate-functionalized polyurethane prepolymers a1);
reacting the polyurethane prepolymer a1) and the isocyanate-reactive compound a2); and dispersing the anionic bio-based polyurethane in water before, during or after the reaction.

The anionic groups comprised in the anionic aqueous bio-based polyurethane dispersion is introduced by the amino-functional compound with an ionic group or ionogenic group in the isocyanate-reactive compound.

The anionic aqueous bio-based polyurethane dispersion can be prepared in one or more stages in a homogeneous phase or, for multistage reactions, partly in the dispersed phase. The anionic aqueous bio-based polyurethane dispersion may be prepared by the prepolymer mixing method, the acetone method or the melt dispersion method, and most preferably the acetone method.

The equivalent ratio of the isocyanate groups to the isocyanate-reactive groups is preferably of 1.05 to 3.5, more preferably 1.1 to 3.0, and most preferably 1.1 to 2.5.

The content of solid ingredients of the anionic aqueous bio-based polyurethane dispersion is preferably of 30% by weight to 50% by weight, relative to the total weight of the anionic aqueous bio-based polyurethane dispersion. The content of solid ingredients is calculated by heating the weighed sample to a constant weight at 125°C and reweighing the sample at the constant weight.

### Component b) bis-diglyceryl polyacyladipate-2

The bis-diglyceryl polyacyladipate-2 is in an amount of 4% by weight to 8% by weight, based on the total weight of the cosmetic composition.

The bis-diglyceryl polyacyladipate-2 is preferably obtained by esterification reaction of a system comprising adipic acid, mixed acids (caprylic acid, capric acid, isostearic acid and hydroxystearic acid) and diglycerol.

The bis-diglyceryl polyacyladipate-2 has preferably the following structure of
The bis-diglyceryl polyac

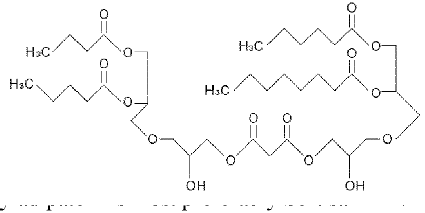

The term "melting point" refers to the temperature at which the solid state is transformed, i.e., melted, to the liquid state. The melting point of the wax of the present invention is obtained by testing according to GB/T 617-2006.

### Component c) wax with a melting point not higher than 65°C

The wax with a melting point not higher than 65°C is preferably a wax with a melting point of 55°C-65°C, most preferably one or more of the following: spermaceti wax, beeswax, sumac wax, aromatic wax and paraffin wax.

The wax with a melting point of not higher than 65° C may be added to the cosmetic composition alone, or may be pre-mixed with other components and then added to the cosmetic composition.

### Component d) wax with a melting point higher than 65°C

The wax with a melting point higher than 65°C is preferably a wax with a melting point of 68°C-88°C, most preferably one or more of the following: carnauba wax, candelilla wax, microcrystalline wax and ozokerite wax.

The wax with a melting point of higher than 65° C may be added to the cosmetic composition alone, or may be pre-mixed with other components and then added to the cosmetic composition.

The wax with a melting point not higher than 65°C and the wax with a melting point higher than 65°C have a weight sum of most preferably 5% by weight to 10% by weight, based on the total weight of the cosmetic composition.

The wax with a melting point not higher than 65°C and the wax with a melting point higher than 65°C have a weight ratio of 0.1 to 1.5.

### Component e) cosmetically acceptable medium

The cosmetically acceptable medium is a medium that is physically and chemically compatible with the components of the cosmetic composition of the present invention and can be used in cosmetics.

The cosmetically acceptable medium may be in an amount as is well known to those skilled in the art. The cosmetically acceptable medium is most preferably in an amount of 40% by weight to 80% by weight, based on the total weight of the cosmetic composition.

The cosmetically acceptable medium is preferably one or more of the following: water, thickeners, oils, emulsifiers, preservatives, humectants, fillers, pigments and film-forming agents.

The water is preferably deionized water.

The thickener is preferably one or more of the following: acrylics, celluloses, diatomaceous earth, carbomers, starches, gelatin, sodium alginate, casein, guar gum, chitosan, acacia Senegal gum, xanthan gum, soy protein gum, bentonite, hectorite, lanolin and derivatives thereof, fatty alcohols and behenate esters, most preferably one or more of the following: xanthan gum and behenates.

The thickener is preferably in an amount of 0% by weight to 10% by weight, based on the total weight of the cosmetic composition.

The emulsifier is preferably one or more of the following: carboxylate , sulfate , sulfonate , amine derivatives, alkyl ethers, stearyl esters, polyoxyethylene ethers, polyoxypropylene ethers, olivate esters, and glutamate, most preferably one or more of the following: stearyl esters, olivate esters, and glutamateacids.

The emulsifier is preferably in an amount of 0% by weight to 10% by weight, based on the total weight of the cosmetic composition.

The oil is preferably one or more of the following: vegetable oils, animal oils, mineral oils and synthetic oils, more preferably one or more of the following: olive oil, coconut oil, castor oil, cottonseed oil, soybean oil, sesame oil, almond oil, peanut oil, corn oil, rice bran oil, tea seed oil, sea buckthorn oil, avocado oil, kukui nut oil, argan oil, walnut oil, cocoa butter, mink oil, egg yolk oil, lanolin oil, lecithin, squalane, lanolin derivatives, polysiloxanes, fatty acids, fatty alcohols, fatty acid esters and vaseline, most preferably one or more of the following: olive oil, coconut oil, castor oil, cottonseed oil, soybean oil, sesame oil, almond oil, peanut oil, corn oil, rice bran oil, tea seed oil, sea buckthorn oil, avocado oil, kukui nut oil, argan oil, walnut oil, cocoa butter, squalane of vegetable origin, lanolin derivative, fatty acids, fatty alcohols and fatty acid esters.

The oil is preferably in an amount of 0% by weight to 10% by weight, based on the total weight of the cosmetic composition.

The preservative is preferably one or more of the following: alcohols, formaldehyde donors, aldehyde derivatives, benzoic acid, benzoic acid derivatives, sodium hydroxymethylglycinate and p-hydroxyacetophenone, more preferably a compound of caprylhydroxamic acid, 1,2-hexanediol and propanediol, most preferably Spectrastat PHL.

The preservative is preferably in an amount of 0% by weight to 2% by weight, most preferably 0.5% by weight to 2% by weight, based on the total weight of the cosmetic composition.

The humectant is preferably one or more of the following: polyols, amides, lactic acid, sodium lactate, sodium pyrrolidone carboxylate, glucose esters, collagen, chitin derivatives and chitosan, more preferably polyols, most preferably one or more of the following: sorbitol and propylene glycol.

The humectant is preferably in an amount of 0% by weight to 10% by weight, based on the total weight of the cosmetic composition.

The filler is preferably one or more of the following: organic powder fillers and inorganic powder fillers.

The inorganic powder filler is preferably one or more of the following: silicon dioxide, diatomaceous earth, layered silicate powder, precipitated calcium carbonate, phosphate and aluminum hydroxide. The layered silicate powder is preferably one or more of the following: kaolin, bentonite, talc and mica powder.

The organic powder filler is preferably one or more of the following: cellulose, nylon powder, polyethylene powder, polystyrene powder, polytetrafluoroethylene powder, synthetic wax micropowder, zinc stearate and magnesium stearate.

The filler is preferably in an amount of 0% by weight to 5% by weight, based on the total weight of the cosmetic composition.

The pigment is preferably one or more of the following: iron oxide, carbon black, mica and colorants.

The pigment is preferably in an amount of 0% by weight to 15% by weight, based on the total weight of the cosmetic composition.

The film-forming agent is preferably one or more of the following: acrylate polymers, silicone polymers, vinyl acetate polymers and vinylpyrrolidone polymers.

### Cosmetic composition

The cosmetic composition may be a mascara. **Examples**

Unless defined otherwise, all technical and scientific terms used herein have the same meaning as commonly understood by those skilled in the art. When the definition of a term in this specification conflicts with the meanings commonly understood by those skilled in the art, the definition described herein shall prevail.

Unless indicated otherwise, all numbers expressing quantities of ingredients, reaction conditions and the like used in the specification and claims are to be understood as being modified by the term "about". Accordingly, the numerical parameters set forth herein are approximations that can vary depending upon the desired properties to be obtained, unless indicated to the contrary.

The wording "and/or" used herein refers to one or all of the cited elements.

The wordings "include" and "comprise" used herein cover the situations in which only the mentioned elements are present and the situations in which other elements not mentioned are present in addition to the mentioned elements.

All percentages in the present invention are weight percentage, and based on the amount of the cosmetic composition of the present invention being 100% by weight, unless otherwise stated.

The number-average molecular weight is determined by gel permeation chromatography (GPC) according to GB/T 21863-2008 "Gel permeation chromatography (GPC) using tetrahydrofuran as the eluent".

The analysis and measurement in the present invention are performed at 23±2°C, unless otherwise stated.

The solid content of the aqueous polyurethane dispersion is calculated by heating the weighed sample to a constant weight at 125°C and reweighing the sample at the constant weight.

### Raw materials and agents

Propanediol : reagent grade.
Sorbitol: reagent grade.
Iron oxide black: INCI name: iron oxide, available from Sensient Unipure Black989.
Keltrol CG-T: INCI: xanthan gum, available from CP Kelco.
Emulium Dolcea MB: INCI name: cetearyl alcohol (and) glyceryl stearate (and) jojoba esters (and) helianthus annuus (sunflower) seed wax (and) sodium stearoyl glutamate (and) water (and) polyglycerin-3, available from Gattefossé.
Olivem^{®} 1000: INCI name: cetearyl olivate (and) sorbitan olivate, available from Hallstar. Cutina^{®} GMS-SE: INCI name: glyceryl stearate SE, available from BASF.
Softisan 649: INCI name: bis-diglyceryl polyacyladipate-2, available from Cremer Care. Compritol 888 CG ATO: INCI name: glyceryl behenate, available from Gattefossé.
8044-1: INCI name: cera alba with a melting point of 63-65°C, available from TerWax. Candelillawax Refined In Pastilles/Flakes: INCI name: candelilla wax with a melting point of 69-73°C, available from TerWax.
Carnauba Wax T1 & T3: INCI name: copernicia cerifera (carnauba) wax with a melting point of 82-85°C, available from TerWax.
Spectrastat PHL: INCI name: caprylhydroxamic acid and 1,2-hexanediol and propanediol, available from INOLEX.
Polyurethane-99: aqueous bio-based polyurethane dispersion, supplied as an aqueous dispersion with a solid content of 40% by weight, available from Covestro.
PB 680: INCI name: hydrogenated polyisobutylene, Daelim Corporation, Korea.
Myritol 318: caprylic/capric triglyceride, available from BASF.
#1 Polyurethane-35: Baycusan C1004/ 1, available from Covestro.
Polyurethane-48: Baycusan C1008/ 1, available from Covestro.
#2 Polyurethane-35: Baycusan C1010, available from Covestro.
SYNTRAN^{®} 5778: INCI name: acrylates/ethylhexyl acrylate copolymer (and) acrylates /dimethylaminoethyl methacrylate copolymer with a solid content of 40% by weight, available from Zschimmer & Schwarz.

### Preparation of the cosmetic composition

According to the components listed in the table, propanediol and xanthan gum were charged into a beaker and dispersed evenly. Deionized water was added and it was stirred until transparent. Then, sorbitol was dissolved and added to the beaker, and stirred evenly to obtain the phase A. All components of the phase B except the iron oxide black were completely melted at 80°C. The iron oxide black was added and stirred evenly to obtain the phase B. The components of the phase C were mixed to obtain the phase C. The phase A was heated to 80°C. Then, the phase B was slowly added to the phase A, and it was stirred rapidly for 5 minutes and then homogenized at 7000 rpm for 10 minutes. After cooling to 45°C, the phase C was added to obtain the cosmetic composition.

### Performance tests

### 1.Smoothness

The eyelashes were curled with an eyelash curler. The cosmetic composition was brushed 10 times in a Z-shape from the root to the tail of eyelashes. According to the smooth feeling of the composition on the eyelashes, it was scored as 1-5 points from low to high. 4 points and above were regarded as qualified. 1 point: the composition was difficult to be brushed on the eyelashes, and it was very dry and rough to brush; 2 points: the composition could be brushed on the eyelashes, but the brushing was not smooth; 3 points: the composition could be brushed on the eyelashes, but the brushing was not very smooth; 4 points: the composition could be brushed smoothly on the eyelashes; 5 points: the composition could be brushed very smoothly on the eyelashes.

### 2. Volume

The false eyelashes were fixed on a cylinder with double-sided adhesive tape. The mascara was brushed 10 times in a Z-shape from top to bottom, and then 10 times from bottom to top. The volume was evaluated according to the amount of the composition on the eyelashes. It was recorded as 1-5 points from low to high, and 4 points and above were regarded as qualified. 1 point: there was no change in the eyelashes compared to those before brushing; 2 points: the eyelashes were slightly changed compared to those before brushing, but not obviously; 3 points: the eyelashes became slightly thicker and darker than those before the brushing; 4 points: the eyelashes became obviously thicker and darker than those before the brushing; 5 points: the eyelashes became very thick and dark compared to those before brushing.

### 3. Natural look

The cosmetic composition was brushed 10 times in a Z-shape from the root to the tail of eyelashes. It was observed for caking and stickiness on the eyelashes, in which the caking refers to the sudden curve at the tail of eyelashes or the sudden thickening of the eyelashes; and sticking refers to the phenomenon in which more than five eyelashes were bonded together into a bundle. Scoring was based on the total number of eyelashes that were curved at the tail, thickened suddenly and adhered together into a bundle, and it was recorded as 1-5 points from low to high, and 4 points and above were regarded as qualified. 1 point: the total number was more than 30; 2 points: the total number was more than 22 and not more than 30; 3 points: the total number was more than 18 and not more than 22; 4 points: the total number was more than 6 and not more than 18; 5 points: the total number was not more than 6.

### 4. Instant curling

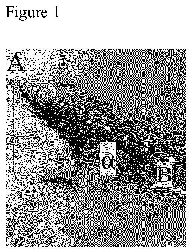

The cosmetic composition was brushed 10 times in a Z-shape from the root to the tail of eyelashes. Photos were taken with fixed shooting angle from a side of the eye. As shown in the figure 1, the tip of eyelashes on the same plane as that of the maximum intersecting surface of the eyeball was taken as point A, and the corner of the eye was taken as point B, and the crossing angle parallel to the picture was made as α. The instant curling degree was defined according to the size of α, wherein the curling degree was recorded as 1-5 points from small to large, and a score of 5 points was regarded as qualified. 1 point: instant curling α was less than 24°; 2 points: instant curling α was not less than 24° and less than 26°; 3 points: instant curling α was not less than 26° and less than 31°; 4 points: instant curling α was not less than 31° and less than 34°; 5 points: instant curling α was not less than 34°.

### 5. Long-lasting curling

The cosmetic composition was brushed 10 times in a Z-shape from the root to the tail of eyelashes. After 8 hours, the long-lasting curling of eyelashes was observed, wherein the long-lasting curling α was marked with reference to the method for the instant curling α. The angle reduction rate was calculated: angle reduction rate= (instant curling α - long-lasting curling α)/instant curling α.

The angle reduction rate was recorded as 1-5 points from poor to good, and 3 points and above were regarded as qualified. 1 point: the angle reduction rate was more than 30%; 2 points: the angle reduction rate was more than 20% and not more than 30%; 3 points: the angle reduction rate was more than 10% and not more than 20%; 4 points: the angle reduction rate was more than 3% and not more than 10%; 5 points: the angle reduction rate was not more than 3%.

### 6. Smudge resistance

The cosmetic composition was brushed 10 times in a Z-shape from the root to the tail of eyelashes. After 8 hours, black particles around the eye were observed. It was scored as 1-5 points from low to high, and a score of 5 points was regarded as qualified. 1 point: the number of black particles around the eye was more than 20; 2 points: the number of black particles around the eye was more than 8 but not more than 20; 3 points: the number of black particles around the eye was more than 4 and not more than 8; 4 points: the number of black particles around the eye was more than 1 but not more than 4; 5 points: the number of particles around the eyes was not more than 1.

### 7. Easy removal

The false eyelashes were fixed on a cylinder with double-sided adhesive tape. The cosmetic composition was brushed 10 times in a Z-shape from top to bottom, and then 10 times from bottom to top. After the composition was completely dry, the false eyelashes were taken off. A cotton pad was wetted with water at 45°C and the false eyelashes were put on the cotton pad. The cotton pad was folded in half, and then the false eyelashes were wiped 10 times from left to right. The composition residue on the false eyelashes was observed and it was scored as 1-5 points from low to high, and 4 points and above were regarded as qualified. 1 point: <20% of the mascara was removed by the cotton pad; 2 points: not less than 20% and less than 40% of the mascara was removed by the cotton pad; 3 points: not less than 40% and less than 60% of the mascara was removed by the cotton pad; 4 points: no less than 60% and less than 80% of the mascara was removed by the cotton pad; 5 points: no less than 80% of the mascara was removed by the cotton pad.

### Cosmetic compositions of Examples and Comparative Examples

Table 1 shows the compositions and results of the performance tests for the cosmetic compositions of Examples 1-5 of the present invention and Comparative Examples 1-15.

The eyelashes brushed with the cosmetic compositions of Examples 1-5 had properties of good smoothness, volume, natural look, instant curling, long-lasting curling, smudge resistance and easy removal.

The cosmetic compositions of Comparative Examples 1 to 3 comprised bis-diglyceryl polyacyladipate-2 in an amount of 0% by weight, 2% by weight or 10% by weight respectively, and the brushed eyelashes with them could not have volume, instant curling and natural look at the same time.

The cosmetic compositions of Comparative Examples 2 and 3 comprised the wax in an amount of less than 4% by weight or greater than 10% by weight, and the brushed eyelashes with them could not have natural look and instant curling at the same time.

The cosmetic compositions of Comparative Examples 4 and 5 comprised the caprylic/capric triglyceride or the hydrogenated polyisobutene, and the brushed eyelashes with them could not have smoothness, volume, natural look, and instant curling at the same time.

The cosmetic compositions of Comparative examples 6 to 8 comprised two waxes of component c and component d in a weight ratio of less than 0.1 or greater than 1.5, and the brushed eyelashes with them could not have natural look, instant curling, and smudge resistance at the same time.

The cosmetic compositions of Comparative Examples 9 to 11 comprised an aqueous bio-based polyurethane dispersion with a solid content of less than 3 % by weight or greater than 9 % by weight, based on the total weight of the cosmetic composition, and the brushed eyelashes with them could not have instant curling, smudge resistance, smoothness and natural look at the same time.

The cosmetic compositions of Comparative Examples 12 to 14 comprised an aqueous polyurethane dispersion that was not bio-based, and the instant curling of the brushed eyelashes with them was regarded as unqualified.

The cosmetic composition of Comparative Example 15 comprised no aqueous polyurethane dispersion, and the brushed eyelashes with it could not have properties of smoothness, instant curling and easy removal at the same time.

**Table 1: Compositions and results of performance tests for the cosmetic compositions of Examples 1-5 of the present invention and Comparative Examples 1-15**

| Component | Trade name | Ex. 1 | Ex. 2 | Ex. 3 | Ex. 4 | Ex. 5 | Comp. Ex. 1 | Comp. Ex. 2 | Comp. Ex. 3 | Comp. Ex. 4 | Comp. Ex. 5 | Comp. Ex. 6 | Comp. Ex. 7 | Comp. Ex. 8 | Comp. Ex. 9 | Comp. Ex. 10 | Comp. Ex. 11 | Comp. Ex. 12 | Comp. Ex. 13 | Comp. Ex. 14 | Comp. Ex. 15 |
|---|---|---|---|---|---|---|---|---|---|---|---|---|---|---|---|---|---|---|---|---|---|
| Phase A | Deionized water /g | to 100 | to 100 | to 100 | to 100 | to 100 | to 100 | to 100 | to 100 | to 100 | to 100 | to 100 | to 100 | to 100 | to 100 | to 100 | to 100 | to 100 | to 100 | to 100 | to 100 |
| | Sorbitol /g | 2.00 | 2.00 | 2.00 | 2.00 | 2.00 | 2.00 | 2.00 | 2.00 | 2.00 | 2.00 | 2.00 | 2.00 | 2.00 | 2.00 | 2.00 | 2.00 | 2.00 | 2.00 | 2.00 | 2.00 |
| | propanediol /g | 4.00 | 4.00 | 4.00 | 4.00 | 4.00 | 4.00 | 4.00 | 4.00 | 4.00 | 4.00 | 4.00 | 4.00 | 4.00 | 4.00 | 4.00 | 4.00 | 4.00 | 4.00 | 4.00 | 4.00 |
| | Keltrol CG-T /g | 1.20 | 1.20 | 1.20 | 1.20 | 1.20 | 1.20 | 1.20 | 1.20 | 1.20 | 1.20 | 1.20 | 1.20 | 1.20 | 1.20 | 1.20 | 1.20 | 1.20 | 1.20 | 1.20 | 1.20 |
| Phase B | Iron oxide black /g | 10.0 0 | 10.0 0 | 10.0 0 | 10.0 0 | 10.0 0 | 10.00 | 10.00 | 10.00 | 10.00 | 10.00 | 10.00 | 10.00 | 10.00 | 10.00 | 10.00 | 10.00 | 10.00 | 10.00 | 10.00 | 10.00 |
| | Emulium Dolcea MB /g | 2.00 | 2.00 | 2.00 | 2.00 | 2.00 | 2.00 | 2.00 | 2.00 | 2.00 | 2.00 | 2.00 | 2.00 | 2.00 | 2.00 | 2.00 | 2.00 | 2.00 | 2.00 | 2.00 | 2.00 |
| | Olivem^{®} 10 00 /g | 3.00 | 3.00 | 3.00 | 3.00 | 3.00 | 3.00 | 3.00 | 3.00 | 3.00 | 3.00 | 3.00 | 3.00 | 3.00 | 3.00 | 3.00 | 3.00 | 3.00 | 3.00 | 3.00 | 3.00 |
| | Cutina^{®} GM S-SE /g | 3.00 | 3.00 | 3.00 | 3.00 | 3.00 | 3.00 | 3.00 | 3.00 | 3.00 | 3.00 | 3.00 | 3.00 | 3.00 | 3.00 | 3.00 | 3.00 | 3.00 | 3.00 | 3.00 | 3.00 |
| | Softisan 649 /g | 4.00 | 8.00 | 4.00 | 4.00 | 4.00 | 0.00 | 2.00 | 10.00 | | | 4.00 | 4.00 | 4.00 | 4.00 | 4.00 | 4.00 | 4.00 | 4.00 | 4.00 | 4.00 |
| | Myritol 318 /g | | | | | | | | | 4.00 | | | | | | | | | | | |
| | PB 680 /g | | | | | | | | | | 4.00 | | | | | | | | | | |
| | Compritol 888 CG ATO /g | 3.00 | 3.00 | 3.00 | 3.00 | 3.00 | 3.00 | 3.00 | 3.00 | 3.00 | 3.00 | 3.00 | 3.00 | 3.00 | 3.00 | 3.00 | 3.00 | 3.00 | 3.00 | 3.00 | 3.00 |
| | 8044-1 /g | 4.00 | 2.22 | 1.00 | 5.40 | 4.00 | 4.00 | 4.89 | 1.33 | 4.00 | 4.00 | 0.00 | 0.51 | 5.67 | 4.00 | 4.00 | 4.00 | 4.00 | 4.00 | 4.00 | 4.00 |
| | Candelillawa x Refined In Pastilles/Flak es /g | 2.00 | 1.11 | 5.33 | 1.44 | 2.00 | 2.00 | 2.44 | 0.67 | 2.00 | 2.00 | 3.60 | 5.66 | 1.33 | 2.00 | 2.00 | 2.00 | 2.00 | 2.00 | 2.00 | 2.00 |
| | Carnauba Wax T1 & T3 /g | 3.00 | 1.67 | 2.67 | 2.16 | 3.00 | 3.00 | 3.67 | 1.00 | 3.00 | 3.00 | 5.40 | 2.83 | 2.00 | 3.00 | 3.00 | 3.00 | 3.00 | 3.00 | 3.00 | 3.00 |
| Phase C | Spectrastat PHL /g | 1.20 | 1.20 | 1.20 | 1.20 | 1.20 | 1.20 | 1.20 | 1.20 | 1.20 | 1.20 | 1.20 | 1.20 | 1.20 | 1.20 | 1.20 | 1.20 | 1.20 | 1.20 | 1.20 | 1.20 |
| | Polyurethane -99 /g | 20.0 0 | 20.0 0 | 20.0 0 | 20.0 0 | 10.0 0 | 20.00 | 20.00 | 20.00 | 20.00 | 20.00 | 20.0 | 20.0 | 20.0 | 0.00 | 5.00 | 25.00 | | | | |
| | #1 Polyurethane -35 /g | | | | | | | | | | | | | | | | | 19.50 | | | |
| | Polyurethane -48 /g | | | | | | | | | | | | | | | | | | 26.70 | | |
| | #2 Polyurethane -35 /g | | | | | | | | | | | | | | | | | | | 20.00 | |
| | SYNTRAN^{®} 5778 /g | | | | | | | | | | | | | | | | | | | | 20.00 |
| Performance tests | | | | | | | | | | | | | | | | | | | | | |
| Smoothness | | 5 | 5 | 5 | 5 | 5 | 4 | 4 | 5 | 2 | 4 | 5 | 4 | 4 | 5 | 5 | 3 | 5 | 5 | 4 | 3 |
| Volume | | 5 | 5 | 5 | 5 | 5 | 3 | 5 | 4 | 2 | 4 | 4 | 5 | 5 | 4 | 5 | 5 | 4 | 5 | 4 | 4 |
| Natural look | | 5 | 5 | 5 | 5 | 4 | 5 | 3 | 3 | 2 | 5 | 3 | 5 | 3 | 5 | 5 | 2 | 5 | 4 | 4 | 4 |
| Instant curling | | 5 | 5 | 5 | 5 | 5 | 2 | 5 | 4 | 2 | 3 | 4 | 4 | 4 | 3 | 4 | 4 | 4 | 4 | 4 | 4 |
| Long-lasting curling | | 4 | 4 | 4 | 4 | 4 | 4 | 4 | 4 | 3 | 3 | 4 | 4 | 4 | 3 | 4 | 4 | 4 | 4 | 4 | 3 |
| Smudge resistance | | 5 | 5 | 5 | 5 | 5 | 5 | 5 | 5 | 5 | 5 | 4 | 5 | 5 | 3 | 4 | 4 | 5 | 5 | 5 | 5 |
| Easy removal | | 5 | 5 | 5 | 5 | 5 | 5 | 5 | 5 | 5 | 5 | 5 | 5 | 5 | 5 | 5 | 5 | 5 | 5 | 5 | 3 |
| Note | | | | | | | Rough appeara nce | | | | | | | | | | | | | | |

| | | | | | | | | | | | | | | | | | | | | | |
|---|---|---|---|---|---|---|---|---|---|---|---|---|---|---|---|---|---|---|---|---|---|
| Note: Component unit: g; the above weights all including the weight of carriers. | | | | | | | | | | | | | | | | | | | | | |

Those skilled in the art will readily understand that the present invention is not limited to the foregoing details, and can be implemented in other specific forms without departing from the spirit or main characteristics of the present invention. Therefore, the examples should be regarded as illustrative rather than restrictive from any point of view, so that the scope of the present invention is illustrated by the claims rather than the foregoing description. Therefore any change, as long as it falls into the meaning and scope of equivalents of the claims, shall be regarded as belonging to the present invention.

## Claims

1. A cosmetic composition, comprising the following components:
a) at least an aqueous bio-based polyurethane dispersion;
b) at least a bis-diglyceryl polyacyladipate-2;
c) at least a wax with a melting point not higher than 65°C;
d) at least a wax with a melting point higher than 65°C; and
e) optionally, a cosmetically acceptable medium;
wherein the aqueous bio-based polyurethane dispersion has a solid content of 3% by weight to 9% by weight, and the bis-diglyceryl polyacyladipate-2 is in an amount of 3% by weight to 9% by weight, and the wax with a melting point not higher than 65°C and the wax with a melting point higher than 65°C have a weight sum of 4% by weight to 10% by weight and a weight ratio of 0.1 to 1.5, the above weight percentages being based on the total weight of the cosmetic composition.

2. The cosmetic composition according to claim 1, **characterized in that** the wax with a melting point not higher than 65°C is a wax with a melting point of 55°C-65°C.

3. The cosmetic composition according to claim 1 or 2, **characterized in that** the wax with a melting point higher than 65°C is a wax with a melting point of 68°C-88°C.

4. The cosmetic composition according to any one of claims 1 to 3, **characterized in that** the wax with a melting point not higher than 65°C and the wax with a melting point higher than 65°C have a weight sum of 5% by weight to 10% by weight, based on the total weight of the cosmetic composition.

5. The cosmetic composition according to any one of claims 1 to 4, **characterized in that** the aqueous bio-based polyurethane dispersion is obtained by reacting a system comprising at least a water-insoluble, water-non-dispersible isocyanate-functionalized polyurethane prepolymer a1) and at least an isocyanate-reactive compound a2).

6. The cosmetic composition according to any one of claims 1 to 5, **characterized in that** the aqueous bio-based polyurethane dispersion comprises at least a sulfonic acid group and/or sulfonate salt group.

7. The cosmetic composition according to any one of claims 1 to 6, **characterized in that** the aqueous bio-based polyurethane dispersion has a solid content of 4% by weight to 8% by weight, based on the total weight of the cosmetic composition.

8. The cosmetic composition according to any one of claims 1 to 7, **characterized in that** the bis-diglyceryl polyacyladipate-2 has the following structure of

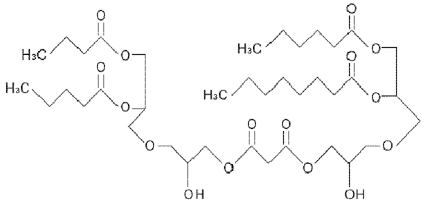

9. The cosmetic composition according to any one of claims 1 to 8, **characterized in that** the bis-diglyceryl polyacyladipate-2 is in an amount of 4% by weight to 8% by weight, based on the total weight of the cosmetic composition.

10. The cosmetic composition according to any one of claims 1 to 9, **characterized in that** the cosmetically acceptable medium is one or more of the following: water, thickeners, oils, emulsifiers, preservatives, and humectants.

11. The cosmetic composition according to any one of claims 1 to 10, **characterized in that** the cosmetic composition is mascara.

12. Method for preparing a cosmetic composition according to any one of claims 1 to 11, including mixing component a) aqueous bio-based polyurethane dispersion, component b) bis-diglyceryl polyacyladipate-2, component c) wax with a melting point not higher than 65°C, component d) wax with a melting point higher than 65°C and component e) optionally a cosmetically acceptable medium to obtain the cosmetic composition.

13. Use of the cosmetic composition according to any one of claims 1 to 11 for applying to keratin fibers for the care or make-up thereof.

14. A mascara comprising the cosmetic composition according to any one of claims 1 to 11.
